# EUROPÄISCHE PATENTSCHRIFT

(11) **EP 3 669 919 B1**
(45) Veröffentlichungstag und Bekanntmachung des Hinweises auf die Patenterteilung: **07.06.2023**
(21) Anmeldenummer: 19217819.2
(22) Anmeldetag: 19.12.2019
(51) Int. Cl.: A61M 16/04

(54) **EINFÜHRHILFE FÜR TRACHEOSTOMIEKANÜLEN**
INSERTION AID FOR TRACHEOSTOMY CANNULAS
AIDE À L'INSERTION POUR CANULES TRACHÉOSTOMIQUES

(30) Priorität: 21.12.2018 DE 102018133449
(43) Veröffentlichungstag der Anmeldung: 24.06.2020
(73) Patentinhaber: Tracoe medical GmbH, 55268 Nieder-Olm (DE)
(72) Erfinder: Schnell, Ralf, 63500 Seligenstadt (DE)
(74) Vertreter: WSL Patentanwälte Partnerschaft mbB

(56) Entgegenhaltungen:
- WO-A1-2009/010070
- DE-A1- 3 430 736
- DE-C2- 3 430 736
- GB-A- 2 160 773
- GB-A- 2 316 321
- US-A1- 2010 059 048
- US-A1- 2011 265 797
- US-A1- 2011 290 245

## Beschreibung

Die vorliegende Erfindung betrifft eine Einführhilfe für eine Tracheostomiekanüle, mit einem durch die Tracheostomiekanüle hindurchführbaren und im Gebrauch aus dem distalen Ende der Tracheostomiekanüle herausragenden Kopf.

Bei einer Tracheotomie wird unterhalb des Kehlkopfs, typischerweise zwischen der 2. und 3. bzw. zwischen der 3. und 4. Knorpelspange der Luftröhre ein künstlicher Zugang zur Luftröhre (Trachea) eines Patienten geschaffen. In dieses sog. Tracheostoma wird eine Tracheostomiekanüle eingesetzt, die einerseits das Stoma offenhalten und andererseits eine Atmung durch die Tracheostomiekanüle ermöglichen soll. Sowohl für die erstmalige Einführung einer Tracheostomiekanüle in das frisch angelegte Tracheostoma, als auch für das wiederholte Einsetzen einer Tracheostomiekanüle bedarf es einer Einführhilfe, die das Einsetzen einer Tracheostomiekanüle erleichtert.

Der aus dem distalen Ende der Tracheostomiekanüle hervorstehende Kopf der Einführhilfe füllt die distale Kanülenöffnung im Wesentlichen vollständig aus. Der Kopf der Einführhilfe soll einen möglichst glatten Übergang zu dem Kanülenrohr der Tracheostomiekanüle bilden, sodass die Tracheostomiekanüle möglichst leicht durch den mit der Einführhilfe geschaffenen Durchgang durch die Gewebeschichten entlang des Tracheostomas gleiten kann, ohne hängen zu bleiben. Da der Kopf der Einführhilfe das distale Ende der Tracheostomiekanüle nahezu vollständig ausfüllt, kann weitestgehend ausgeschlossen werden, dass Gewebe oder Sekret während der Einführung der Tracheostomiekanüle in einen Spalt zwischen der Tracheostomiekanüle und dem Kopf der Einführhilfe in das Kanülenrohr gelangen kann. Nach der Einführung der Tracheostomiekanüle in das Tracheostoma wird die Einführhilfe in die proximale Richtung aus der Tracheostomiekanüle herausgezogen, um das Lumen der Tracheostomiekanüle freizugeben.

Einführhilfen, wie sie aus der US 4,246,897 A, US 5,546,937 A, EP 1 044 701 A1, EP 0 824 928 B1, US 5,222,487 A und US 2015 012264 A1 bekannt sind, weisen immer eine Stufe zwischen dem Kopf der Einführhilfe und dem distalen Ende der Tracheostomiekanüle auf. Die Höhe der Stufe entspricht dabei der Summe der Wandstärke des Kanülenrohres und dem radialen Abstand zwischen der Einführhilfe und dem Innendurchmesser der Kanüle. Die Stufe kann bei der Einführung der Tracheostomiekanüle an eine Knorpelspange, Gewebeschichten oder Vorsprünge innerhalb der Trachea anstoßen, und so eine weitergehende Einführung verhindern oder zumindest erschweren. Dies umso mehr, je größer die Stufe im Vergleich zu dem Außendurchmesser der Tracheostomiekanüle ist.

Auch das Patentdokument DE3430736A1 wird anerkannt.

Die Spitzen der bekannten Einführhilfen bestehen zumeist aus einem auch im Vergleich zu der Trachealschleimhaut starren Material, sodass die Spitze beim Auftreffen die Trachealwand beschädigen kann. Insbesondere für Neugeborene und Kleinkinder besteht ein Bedarf an Einführhilfen, die das Risiko von traumatischen Einführungen von Tracheostomiekanülen mindern. Je geringer der Außendurchmesser des Kopfs der Einführhilfe desto kleiner ist die auf die Trachealwand auftreffende Fläche des Kopf und desto größer ist die Gefahr von Verletzungen.

Eine modifizierte Einführhilfe ist in der europäischen Patentanmeldung EP 1 893 272 B1 beschrieben. Die Einführhilfe weist an ihrem distalen Ende einen Kopf auf, der durch die in das Tracheostoma einzusetzende Tracheostomiekanüle hindurchführbar ist und der im Gebrauch aus der an dem distalen Ende der Tracheostomiekanüle angeordneten Öffnung herausragt. Der Kopf bildet eine konische Spitze, die in einem ersten Zustand einen kleinen Basisdurchmesser und in einem zweiten Zustand einen großen Basisdurchmesser aufweist. Der kleine Basisdurchmesser entspricht höchstens dem Innendurchmesser der Tracheostomiekanüle, sodass der Kopf in seinem ersten Zustand durch die Tracheostomiekanüle hindurchgeführt werden kann. Ragt der Kopf aus der distalen Öffnung der Tracheostomiekanüle heraus, kann der Kopf in seinen zweiten Zustand gebracht werden. In dem zweiten Zustand ist der große Basisdurchmesser größer als der Innendurchmesser der Tracheostomiekanüle und entspricht vorzugsweise in etwa dem Außendurchmesser der Tracheostomiekanüle. Der Kopf mit seinem im zweiten Zustand großen Basisdurchmesser dient der Aufweitung eines Tracheostoma beim Einführen einer Tracheostomiekanüle, die hinter dem Kopf die Einführhilfe umschließt. Ein proximales Ende der Einführhilfe ragt aus dem proximalen Ende der Tracheostomiekanüle heraus und ermöglicht eine Einwirkung auf den Kopf, und um die Einführhilfe im ersten Zustand durch die Tracheostomiekanüle hindurch zu schieben und ein Herausziehen aus nach dem Einführen in ein Tracheostoma zu ermöglichen.

Der Kopf in Form einer konischen Spitze besteht aus einem inneren Kern und einer äußeren Hülse, die am oder in der Nähe des distalen Endes der Spitze miteinander verbunden sind. Der Kern ist weiter derart mit einem Zugelement verbunden, dass durch ein Ziehen des Zugelements entgegen der durch die konische Spitze vorgegebenen Richtung die sich am distalen Ende der Tracheostomiekanüle abstützende Hülse von ihrem Ansatz an den Kern umstülpt und sich aufgrund ihrer elastischen Vorspannung in radialer Richtung zusammenzieht bis ihr Außendurchmesser kleiner als der Innendurchmesser der Tracheostomiekanüle ist. Auf diese Weise kann der Kopf von seinem zweiten Zustand in seinen ersten Zustand überführt werden. Entsprechend kann durch ein Betätigen des Zugelementes in Richtung des distalen Endes der Tracheostomiekanüle der Kopf von seinem ersten Zustand in seinen zweiten Zustand überführt werden.

Da der Kopf in seinem zweiten Zustand in etwa den Außendurchmesser der Kanüle hat und der Aufweitung des Tracheostomas dient, wird mit dem Kopf in seinem zweiten Zustand eine entsprechende Kraft auf die Ränder des Tracheostomas ausgeübt. Für einen Benutzer einer solchen Einführhilfe ist jedoch nicht ersichtlich, wodurch genau der zum Einbringen der Einführhilfe erforderliche Kraftaufwand hervorgerufen wird, zum Beispiel durch den Widerstand der Ränder des Tracheostomas oder dadurch, dass die Einführhilfe gegen die Trachealspangen oder die dem Tracheostoma gegenüberliegende Trachealwand drückt. Bei der Verwendung herkömmlicher Einführhilfen besteht daher die Gefahr, dass beim Verwenden der Einführhilfe die Trachea beschädigt wird. Insbesondere bei einem plötzlichen Weiten des Tracheostomas kann beim Einbringen der Einführhilfe die Trachea eines Patienten verletzt werden.

Es ist daher eine Aufgabe der vorliegenden Erfindung eine Einführhilfe für eine Tracheostomiekanüle bereitzustellen, die hilft unbeabsichtigte Verletzungen der Trachea eines Patienten beim Einsetzen einer Tracheostomiekanüle besser zu vermeiden.

Diese Aufgabe wird durch eine Einführhilfe der eingangs genannten Art gelöst, die erfindungsgemäß dadurch gekennzeichnet ist, dass der Kopf der Einführhilfe hohl ist und der Hohlraum des Kopfes über einen durch das Lumen der Tracheostomiekanüle verlaufenden Schlauch mit einem variablen Fluidreservoir zum Befüllen und Entleeren des Hohlraums verbunden ist, das im Gebrauch auf der proximalen Seite der Tracheostomiekanüle angeordnet ist, und dass der Kopf eine flexible Hülle aufweist, so dass der Kopf bei gefülltem Hohlraum gegenüber der Längsachse der Tracheostomiekanüle einen maximalen Außendurchmesser annimmt, der größer als der Innendurchmesser der Tracheostomiekanüle ist, und bei entleertem Hohlraum gegenüber der Längsachse der Tracheostomiekanüle einen maximalen Außendurchmesser annimmt, der kleiner ist als der Innendurchmesser der Tracheostomiekanüle. Aufgrund der Flexibilität des Kopfes wird das Risiko von Trachealschäden reduziert, da der Kopf Druck auf die Schleimhaut der Trachea abpolstert.

Der Hohlraum des Kopfes ist, abgesehen von der Verbindung mit dem Schlauch, ein allseitig geschlossener Hohlraum.

Durch Befüllen des Hohlraumes mit einem Fluid aus dem Fluidreservoir kann der Kopf der Einführhilfe auf ein Übermaß gegenüber dem Innendurchmesser der Tracheostomiekanüle ausgedehnt werden. Der maximale Außendurchmesser des im Gebrauch aus der distalen Tracheostomiekanüle herausragenden und mit einem Fluid aus dem Fluidreservoir befüllten Kopfes ist in radialer Richtung der Tracheostomiekanüle größer als der Innendurchmesser der Tracheostomiekanüle. Der vergrößerte, maximale Außendurchmesser verhindert, dass der mit dem Fluid befüllte Kopf während der Einführung der Tracheostomiekanüle versehentlich zurück durch die Tracheostomiekanüle gezogen oder gedrückt werden kann. Durch den vergrößerten, maximalen Außendurchmesser entsteht ein dichter und idealerweise bündiger Übergang zwischen dem Kopf und der Außenseite der Tracheostomiekanüle, der ein Eingreifen oder Eindringen von Gewebe in den Übergang zwischen Kopf und Tracheostomiekanüle verhindert. Gleichzeitig kann der mit Fluid gefüllte Kopf zur Aufweitung eines Tracheostomas bzw. zum Aufspreizen von Gewebeschichten genutzt werden, um die Tracheostomiekanüle leichter durch das Tracheostoma führen zu können.

Der mit Fluid gefüllte Kopf bildet das äußerste distale Ende der Einführhilfe und kann hierfür derart in ein Tracheostoma eingeführt werden, dass die äußere Hülle des ausgedehnten Kopfes gegen die Ränder des Tracheostoma drückt. Der mit Fluid befüllte Kopf weitet beim Durchdringen den Öffnungsquerschnitt des Tracheostomas auf den maximalen Außendurchmesser des mit Fluid befüllten Kopfes der Einführhilfe auf. Der beim Durchstoßen des Tracheostomas auf den Kopf einwirkende Widerstand führt, bedingt durch die flexible, möglichst dünnwandige Hülle, einen Druckanstieg in dem mit Fluid befüllten Hohlraum des Kopfs.

Da der mit Fluid befüllte Hohlraum des Kopfs über den im Lumen der Tracheostomiekanüle angeordneten Schlauch mit dem Fluidreservoir verbunden ist, führt der Druckanstieg in dem mit Fluid befüllten Kopf dazu, dass ein Teil des in dem Hohlraum des Kopfs befindlichen Fluids zurück in das Fluidreservoir drängt. In der Folge des Druckanstiegs in dem Hohlraum des Kopfs steigt auch der Druck in dem variablen Fluidreservoir an. Die in dem variablen Fluidreservoir feststellbare Änderung des Drucks indiziert auf den mit Fluid befüllten Kopf wirkende Kräfte. Beispielsweise kann ein übermäßiger Druckanstieg auf eine drohende Verletzung der Trachea hinweisen, sei es weil der Kopf der Einführhilfe bereits das Tracheostoma durchstoßen hat und gegen die dem Stoma gegenüberliegende Trachealwand drückt oder sei es weil der Kopf der Einführhilfe an einer oder mehreren Knorpelspangen der Trachea festhängt oder sich das Tracheostoma aus sonstigen Gründen nicht bis zum maximalen Außendurchmesser des mit Fluid befüllten Kopfs aufweiten lässt. Wenn der Anwender anhand der Druckänderung bemerkt, dass die Einführhilfe anstößt bzw. sich nicht weiter einführen lässt, kann der Anwender beispielsweise den Einführwinkel variieren oder insgesamt die Einführhilfe drehen, um einen weiteren Druckanstieg zu vermeiden, bei dem eine Verletzung zu befürchten wäre

Strömt das Fluid von dem mit Fluid befüllten Hohlraum zurück in das Fluidreservoir, wird dem Hohlraum Fluid entzogen und die flexible Hülle des Kopfes kehrt weitestgehend in ihren Ursprungszustand zurück. Der maximale Außendurchmesser des Hohlraums mit einer Hülle im Ausgangszustand ist kleiner als der Innendurchmesser der Trachoestomiekanüle, sodass die Einführhilfe aus der proximalen Öffnung der Tracheostomiekanüle heraus- und hindurchgezogen bzw. umgekehrt in die Tracheostomiekanüle eingesetzt werden kann.

Die Begriffe "distal" und "proximal" werden im Sinne der vorliegenden Erfindung aus der Sicht eines behandelnden Arztes verwendet, d.h. das proximale Ende der Tracheostomiekanüle liegt außerhalb des Patientenkörpers, während das distale Ende im eingebrachten Zustand der Tracheostomiekanüle im Inneren der Trachea des Patienten angeordnet ist. Im Sinne der vorliegenden Erfindung ist ein "entleerter" Hohlraum des Kopfes nicht zwingend restlos entleert, vielmehr weist der Begriff "entleert" auf einen soweit geleerten Hohlraum hin, dass die Hülle des Kopfes nicht über den Innendurchmesser der Kanüle hinaus durch das Fluid ausgedehnt ist.

In einer bevorzugten Ausführungsform der vorliegenden Erfindung hat die Hülle eine mittlere Wandstärke in einem Bereich von 5 µm bis 1 mm. Vorzugsweise hat die Hülle eine mittlere Wandstärke von 10 µm bis 500 µm. Eine Hülle wird als flexibel angesehen, wenn sich ihre Außenkontur durch ein Befüllen mit Fluid und ein Entleeren von Fluid verändern lässt, insbesondere in den oben erwähnten ersten und zweiten Zustand.

In einer Ausführungsform bilden das variable Fluidreservoir, der Schlauch und der Kopf ein geschlossenes Volumen. Da das in dem geschlossenen Volumen befindliche Fluid keine andere Ausweichmöglichkeit hat, bedingt eine den Kopf zusammendrückende Kraft, dass das in dem Kopf befindliche Fluid durch den Schlauch in das Fluidreservoir drängt und dort einen Druckanstieg und/oder eine Volumenänderung des Fluidreservoirs bewirkt. Entsprechend bewirkt eine das Volumen des Fluidreservoirs vermindernde Kraft, dass das Fluid aus dem Fluidreservoir durch den Schlauch in den Kopf drängt. Auf diese Weise kann der Au ßendurchmesser des Kopfs gegenüber der Längsachse der Tracheostomiekanüle auf ein Übermaß gebracht und zur Aufweitung eines Tracheostomas verwendet werden. Aufgrund des geschlossenen Volumens führen Druck-, Volumen- und/oder Kraftänderungen in dem Fluidreservoir zu einer unmittelbaren Druck-, Volumen- und/oder Kraftänderung in dem Kopf und umgekehrt. Eine Druck-, Volumen- und/oder Kraftänderung an dem Fluidreservoir kann auf diese Weise als Indikator für die auf den Kopf einwirkenden Kräfte und/oder den Füllzustand des Kopfs dienen.

Die Empfindlichkeit einer Rückmeldung über eine Druck-, Volumen und/oder Kraftänderung kann verbessert werden, wenn das maximale Volumen des variablen Fluidreservoirs nicht mehr als das 0,5-fache bis 5-fache des maximalen Volumen des Kopfes beträgt, wobei vorzugsweise das maximale Volumen des variablen Fluidreservoirs und das maximale Volumen des Kopfes gleich groß sind. Bei einer in etwa gleich großen Fluidaufnahmekapazität von Fluidreservoir und Kopf führt eine Druck-, Volumen- und/oder Kraftänderung in dem Kopf in etwa im gleichen Ausmaß zu einer Druck-, Volumen- und/oder Kraftänderung in dem Fluidreservoir, und umgekehrt, und ist damit von außen festzustellen bzw. zu fühlen. In einer Ausführungsform beträgt das maximale Füllvolumen des Fluidreservoirs von 0,3 ml bis 5 ml und/oder das maximale Füllvolumen des Kopfs von 0,1 ml bis 3 ml.

Vorzugsweise wird der Kopf mit einem Fluid mit einem Druck von 20 mbar bis 100 mbar beaufschlagt. Der mit Fluid befüllte Kopf hat gegenüber der Längsachse der Tracheostomiekanüle einen maximalen Außendurchmesser der größer als der Innendurchmesser der Tracheostomiekanüle ist und vorzugsweise dem Außendurchmesser der Tracheostomiekanüle an ihrem distalen Ende entspricht oder um bis zu 10 %, bevorzugt um bis zu 2 %, größer als der Außendurchmesser der Tracheostomiekanüle an ihrem distalen Ende ist. In einer Draufsicht auf die Stirnseite des distalen Endes der Tracheostomiekanüle verdeckt der maximal mit Fluid befüllte Kopf das distale Ende der Tracheostomiekanüle vollständig.

In einer Ausführungsform sind das Fluidreservoir, der Schlauch und der Kopf derart aufeinander abgestimmt, dass der Kopf bei einer Aufnahme des gesamten Fluid aus dem Fluidreservoir seinen maximalen Außendurchmesser annimmt, der größer als der Innendurchmesser der Tracheostomiekanüle ist und vorzugsweise dem Außendurchmesser der Tracheostomiekanüle an ihrem distalen Ende entspricht oder um bis zu 10 %, bevorzugt um bis zu 2 %, größer als der Außendurchmesser der Tracheostomiekanüle an ihrem distalen Ende ist.

In einer Ausführungsform hat die Tracheostomiekanüle einen Innendurchmesser in einem Bereich 2 mm bis 12 mm, vorzugsweise 2,5 mm oder 11 mm, und einen Außendurchmesser in einem Bereich von 4 mm bis 15 mm, vorzugsweise 4,5 mm oder 14 mm. Der maximale Außendurchmesser des vollständig mit Fluid befüllten Kopfs kann in einer Ausführungsform 2 mm bis 4 mm betragen.

Das Fluidreservoir der Einführhilfe kann beispielsweise ein von Hand zusammendrückbarer, elastischer Ballon sein. Der Ballon kann weiter beispielsweise eine Pilzform, Tellerform, Halbkugelform, Olivenform, Eierform oder Kugelform haben. Beim Zusammenpressen des Ballons oder beim Betätigen des Kolbens wird Fluid aus dem Fluidreservoir über den im Lumen der Tracheostomiekanüle angeordneten Schlauch in den Hohlraum des Kopfes der Einführhilfe geführt, wo die Fluidzunahme eine Ausdehnung des Hohlraums des Kopfes bewirkt. Entsprechend sinkt das Volumen des variablen Fluidreservoirs durch die Fluidabnahme, sodass der Füllstand des Kopfes und damit die Ausdehnung des Außendurchmessers des Kopfes anhand der Volumenabnahme des Fluidreservoirs bestimmt werden können. In einer Ausführungsform ist das Fluid inkompressibel, sodass eine Volumenänderung des Kopfs unmittelbar zu einer identischen Volumenänderung des Fluidreservoirs führt, und umgekehrt. Die auf das Fluidreservoir zum Zusammendrücken des Fluidreservoirs ausgeübte Kraft entspricht der im Gebrauch über den Kopf der Einführhilfe auf das Tracheostoma oder die Trachea ausgeübten Kraft. Durch die Druck- und/oder Volumenveränderung beim Betätigen des Fluidreservoirs bekommt ein Benutzer der Einführhilfe ein direktes Feedback, wie groß der mit dem Kopf der Einführhilfe ausgeübte Druck ist. Dies ermöglicht dem Anwender u.a. durch Variation des Einführwinkels den Weg des geringsten Widerstands zu finden. Der weiche Ballon ist bei einem Kontakt mit der empfindlichen Schleimhaut besonders schonend und hilft Blutungen zu vermeiden bzw. zu reduzieren.

Eine Verwendung von Luft oder Gas als Fluid hat den Vorteil, dass im Falle einer Beschädigung oder Undichtigkeit des Kopfes der Einführhilfe keine Flüssigkeit in die Bronchien des zu behandelnden Patienten gelangen kann. Aufgrund ihrer Kompressibilität unterstützt die Verwendung von Luft oder Gas zusätzlich die polsternden Eigenschaften des Kopfs. Gleichwohl sind Änderungen der auf den Kopf wirkenden Kräfte auch bei der Verwendung von kompressiblen Fluiden an einer Volumenänderung des Fluidreservoirs spürbar.

Die Hülle des Fluidreservoirs kann eine lokale Verstärkung aufweisen, die eine vorbestimmte Faltenbildung beim Zusammenpressen der Hülle des Fluidreservoirs bewirkt. Beispielsweise kann die Hülle des Fluidreservoirs eine Mehrzahl konzentrisch zueinander angeordneter Verstärkungsringe oder Wülste aufweisen, die ein Zusammendrücken der Hülle durch Ausüben eines Drucks auf den Kreismittelpunkt erleichtern. Die Hülle des Fluidreservoirs kann auch eine ergonomische Form oder zumindest einen ergonomischen Abschnitt für das Greifen und/oder Betätigen mit einer Hand aufweisen.

Das proximale Ende der Einführhilfe kann einen Griff, beispielsweise einen sich in radialer Richtung der Einführhilfe erstreckenden Abschnitt oder Flansch aufweisen. Der Griff dient dem einhändigen Greifen der Einführhilfe und erleichtert ein gleichzeitiges Zusammendrücken des Fluidreservoirs. Vorzugsweise ist der Griff derart an dem proximalen Ende der Einführhilfe angeordnet, dass er im Gebrauch mit mindestens zwei Fingern ergriffen werden kann, während mit dem Daumen oder Handballen derselben Hand das Fluidreservoir betätigt werden kann. Der Griff kann auch ein in Umfangsrichtung des Fluidreservoir umlaufender Rand sein.

Als Anhaltspunkt dafür, wie hoch die mit dem Kopf auf ein Tracheostoma oder eine Trachea im Gebrauch der Einführhilfe ausgeübte Kraft ist, kann die Hülle des Fluidreservoirs eine Markierung oder Skala aufweisen, die den im Kopf der Einführhilfe vorliegenden Druck entsprechend der Verformung der Hülle des Fluidreservoir und/oder der Volumenabnahme des Fluidreservoirs anzeigt.

Der Schlauch kann auch ein Volumenstrommesselement und/oder ein Druckmesselement aufweisen. Ein Volumenstrommesselement und/oder ein Druckmesselement helfen den Füllstand des Hohlraums in dem Kopf der Einführhilfe zu bestimmen, einzustellen und/oder zu kontrollieren.

Der das Fluidreservoir und den Kopf verbindende Schlauch kann aus einem härteren Material bestehen als die Hülle des Kopfs. Der das Fluidreservoir und den Kopf verbindende Schlauch ist im Gebrauch der Einführhilfe in dem Lumen der Tracheostomiekanüle angeordnet und dient primär der Durchleitung von Fluid. Ein aus einem hinreichend harten Material hergestellte Schlauch kann zusammen mit der Tracheostomiekanüle oder durch diese hindurch in die distale Richtung geschoben werden, ohne dass sich der Schlauch verformt. Auf diese Weise unterstützt der Schlauch eine Vorwärtsbewegung in Richtung bzw. durch ein Tracheostoma. Gleichzeitig vereinfacht ein solcher Schlauch das Herausziehen der Einführhilfe aus einer Tracheostomiekanüle, wenn der Hohlraum des Kopfes entleert ist.

Der das Fluidreservoir und den Kopf verbindende Schlauch kann ein oder mehrere Abstandhalter aufweisen, mit denen der Schlauch in dem Lumen der Tracheostomiekanüle mittig geführt werden kann. Beispielsweise kann der Schlauch ein oder mehrere radial umlaufende Rippen oder eine Vielzahl in Umfangsrichtung angeordnete Vorsprünge, vorzugsweise in der Nähe des Kopfes, aufweisen, die den Schlauch mittig in dem Lumen der Tracheostomiekanüle halten. Die Abstandhalter verbessern, insbesondere beim Ansetzen der Einführhilfe, die Führungsgenauigkeit der Einführhilfe durch ein Tracheostoma.

Der Schlauch kann Mittel aufweisen, die ihn leichter biegbar machen. Beispielsweise kann der Schlauch Einschnürungen oder Abschnitte mit einer reduzierten Wandstärke aufweisen.

Der im gefüllten Zustand des Kopfes den maximalen Durchmesser definierende Abschnitt des Kopfes kann näher an dem distalen Ende der Tracheostomiekanüle liegen als an dem distalen Ende des Kopfes. Um ein Einreißen der Ränder des Trachestomas zu verhindern, sollte das Tracheostoma möglichst gleichmäßig und allmählich geweitet werden, wobei die maximale Aufweitung des Tracheostoma erst kurz vor der eigentlichen Einführung der Tracheostomiekanüle erreicht werden sollte. Beginnend mit einem im Vergleich zu dem maximalen Außendurchmesser des Kopfs kleinen Außendurchmesser an dem distalen Ende des Kopfs, kann das Tracheostoma bis zum Erreichen des maximalen Außendurchmessers aufgeweitet werden. Der näher an der distalen Tracheostomiekanülenöffnung angeordnete maximale Außendurchmesser hilft zudem einen Teil der Kräfte, die über die Ränder des Tracheostomas auf den Kopf einwirken, auf die Tracheostomiekanüle zu übertragen.

Der flexible Kopf kann im gefüllten Zustand annähernd die Form eines an der Spitze abgerundeten Kegels mit einer Basis, einer Spitze und einer sich von der Basis zur Spitze hin verjüngenden, umlaufenden Seitenwand annehmen, wobei die Basis näher an dem distalen Ende der Tracheostomiekanüle als die Spitze liegt.

In einer Ausführungsform weist der flexible Kopf einen Halsabschnitt auf, der in einem Zustand, in welchem der Kopf aus dem distalen Ende der Tracheostomiekanüle herausragt, zumindest abschnittsweise im Lumen der Tracheostomiekanüle angeordnet ist, wobei der Halsabschnitt bei gefülltem Hohlraum des Kopfs an der das Lumen der Tracheostomiekanüle begrenzenden Wand der Tracheostomiekanüle anliegt. Vorzugsweise liegt der Halsabschnitt bei einem bis zum Maximum gefüllten Hohlraum des Kopfs vollumfänglich an der das Lumen der Tracheostomiekanüle begrenzenden Wand an. Der Halsabschnitt dient bei einem gefüllten Hohlraum des Kopfs der Abdichtung der Tracheostomiekanüle und erhöht die Stabilität des Kopfs.

Um den Kopf in seinem entleerten Zustand einfach aus der Tracheostomiekanüle herausziehen zu können, kann der Kopf lokale Verstärkungen aufweisen, um eine gezielte Faltenbildung des Kopfes zu erzeugen, wenn das Fluid aus dem Kopf abgezogen wird, aufweisen. Darüber hinaus können die lokalen Verstärkungen die Form des Kopfs in seinem befüllten Zustand unterstützen, sodass die auf den Kopf einwirkenden Kräfte den Kopf weniger verformen.

Die lokalen Verstärkungen können beispielsweise in der Seitenwand von der Spitze zur Basis verlaufende Rippen sein. Die in der Seitenwand der Hülle von der Spitze zu der Basis verlaufenden Rippen helfen Kräfte von der Spitze auf die Basis, und über die Basis auf die Tracheostomiekanüle zu übertragen. Die Rippen können an einem die Spitze des Kopfs umgebenden zentralen Verstärkungsring enden, dessen Außendurchmesser kleiner als der Innendurchmesser der Tracheostomiekanüle ist. Da der die Spitze des Kopfs umgebende, zentrale Verstärkungsring einen Außendurchmesser aufweist, der kleiner als der Innendurchmesser der Tracheostomiekanüle ist, kann der Kopf mit dem Verstärkungsring in seinem entleerten Zustand gut durch die Tracheostomiekanüle hindurch bewegt werden. Radial innerhalb des Verstärkungsringes ist die dort ohne Rippen ausgebildete Spitze des Kopfes relativ nachgiebig und leicht verformbar, so dass der behandelnde Arzt eine spürbare Rückmeldung erhält, wenn diese Spitze an die dem Tracheostoma gegenüberliegende Rückwand der Trachea anstößt.

Der das Fluidreservoir und den Kopf verbindende Schlauch kann mit einem Ende in den Hohlraum des Kopfes hineinragen, wobei sich das in den Hohlraum hineinragende Ende des Schlauches in distale Richtung verjüngt und abgerundete Kanten aufweist. Die in den Hohlraum des Kopfes hineinragenden Schlauchabschnitte können helfen einen Teil der auf den Kopf einwirkenden Kräfte aufzunehmen. Sollte sich der Kopf durch die auf ihn im Gebrauch einwirkenden Kräfte derart stark verformen, dass die flexible, dünnwandige Hülle des Kopfs gegen die in den Kopf hineinragenden Schlauchabschnitte drückt, verhindert de Schlauchabschnitt eine weitergehende Verformung des Kopfs. Der sich in eine distale Richtung verjüngende Schlauchabschnitt kann bei einem niedrigen Füllstand des Kopfs als ein durch das Material des Kopfs verhüllter Dorn der Führung in ein Tracheostoma und/oder einer initialen Aufweitung eines Tracheostomas dienen.

Die flexible, dünnwandige Hülle des Kopfes kann aus einem Elastomer bestehen, wobei das Elastomer vorzugsweise eine Shore-A-Härte in einem Bereich von 5 bis 70 aufweist. Die Elastizität des Materials der Hülle bewirkt, dass die dünnwandige Hülle nach einer Entleerung des Kopfs sich zusammenzieht und in ihren entleerten Ausgangszustand zurückkehrt. Auf diese Weise kann der Kopf der Einführhilfe einfach durch die Tracheostomiekanüle zurückgezogen werden. Die flexible, dünnwandige Hülle aus einem Elastomer hat vorzugsweise eine mittlere Wandstärke in einem Bereich von 100 µm bis 1 mm. Die Hülle des Kopfes kann beispielsweise ein thermoplastisches Elastomer, wie beispielsweise ein thermoplastisches Styrolblockcopolymer (SEBS), thermoplastisches Polyamidelastomer, Polyurethan, Ethylen-Vinylacetat-Copolymer (EVA) oder Silikon sein

Alternativ kann die flexible, dünnwandige Hülle des Kopfes aus einem im relativen Druckbereich bis zum 100 mbar unelastischen Material bestehen. Eine unelastische Hülle hat in dem befüllten Zustand des Kopfs ein Übermaß gegenüber dem Innendurchmesser der Tracheostomiekanüle. Allerdings kann die Hülle in dem entleerten Zustand des Kopfs derart zusammengefaltet bzw. zusammengezogen werden, dass die leere Hülle durch die Tracheostomiekanüle gezogen werden kann. Sowohl in einem befüllten als auch in einem entleerten Zustand übt die unelastische Hülle keine äußeren Kräften entgegen wirkende Rückstellkraft aus, so dass auf die Hülle wirkende Kräfte unmittelbar und unverfälscht auf das Fluid und in das Fluidreservoir übertragen werden. Die flexible, dünnwandige Hülle aus einem unelastischen Material hat vorzugsweise eine mittlere Wandstärke in einem Bereich von 10 µm bis 250 µm.

Die Endabschnitte, der flexiblen dünnwandigen Hülle sind typischerweise über ihre Länge und ihren Umfang hinweg mit der Außenwand des Schlauches dicht verklebt oder verschweißt.

Der Kopf kann einen Kanal zum Hindurchführen eines Seldingerdrahts und/oder eines Führungskatheters aufweisen. Beispielsweise weist der Kopf in seinem befüllten Zustand eine Torusform auf, durch dessen zentrale Öffnung ein Seldingerdraht und/oder ein Führungskatheter geführt werden kann.

Im Gebrauch wird die erfindungsgemäße Einführhilfe in der Regel zusammen mit einer Tracheostomiekanüle verwendet, wobei die Einführhilfe und die Tracheostomiekanüle getrennt voneinander vermarktet werden können. Die Einführhilfe ist als Ergänzung für eine Tracheostomiekanüle anzusehen.

Die vorliegende Erfindung betrifft daher auch ein Set mit einer Tracheostomiekanüle und einer erfindungsgemäßen Einführhilfe, wobei die Einführhilfe und die Tracheostomiekanüle derart dimensioniert sind, dass der Kopf der Einführhilfe und der den Kopf mit dem Fluidreservoir verbindende Schlauch durch die Tracheostomiekanüle hindurchführbar sind, sodass der Kopf aus der distalen Öffnung der Tracheostomiekanüle herausragt und das Fluidreservoir auf der proximalen Seite der Tracheostomiekanüle angeordnet ist, und wobei der Kopf bei gefülltem Hohlraum einen maximalen Außendurchmesser annimmt, der größer als der Innendurchmesser der Tracheostomiekanüle ist, und bei entleertem Hohlraum einen maximalen Außendurchmesser annehmen kann, der kleiner ist als der Innendurchmesser der Tracheostomiekanüle.

Es versteht sich, dass alle zuvor und nachfolgend beschriebenen Merkmale der Einführhilfe auch als im Zusammenhang mit einem Set aus einer erfindungsgemäßen Einführhilfe und einer Tracheostomiekanüle offenbart gelten sollen, auch wenn die Merkmale nur im Zusammenhang mit der Einführhilfe beschrieben sind. Entsprechend gelten alle Merkmale der Einführhilfe, die zuvor oder nachfolgend im Zusammenhang mit dem erfindungsgemäßen Set beschrieben sind, auch als im Zusammenhang mit der erfindungsgemäßen Einführhilfe in Alleinstellung offenbart.

Weitere Vorteile, Merkmale und Anwendungsmöglichkeiten der vorliegenden Erfindung werden anhand der vorliegenden Beschreibung einer Ausführungsform und der dazugehörigen Figuren deutlich, wobei gleiche Bezugszeichen auf gleiche Elemente verweisen. Es zeigen:
- Figur 1a: einen Längsschnitt durch ein Set mit einer erfindungsgemäßen Einführhilfe und einer Tracheostomiekanüle gemäß einer Ausführungsform der Erfindung mit einem entleerten Kopf;
- Figur 1b: eine Draufsicht von oben auf das Fluidreservoir aus Figur 1a,
- Figur 2a: einen Querschnitt durch das Set aus den Figuren 1a und 1b mit einem mit Fluid befüllten Kopf,
- Figur 2b: eine Draufsicht von oben auf das Fluidreservoir aus der Figur 1b,
- Figur 3a: eine Detailansicht eines Kopfes einer Einführhilfe gemäß einer weiteren Ausführungsform der vorliegenden Erfindung, und
- Figur 3b: eine Detailansicht eines Querschnitts durch ein Set gemäß der Ausführungsform aus Figur 3a.

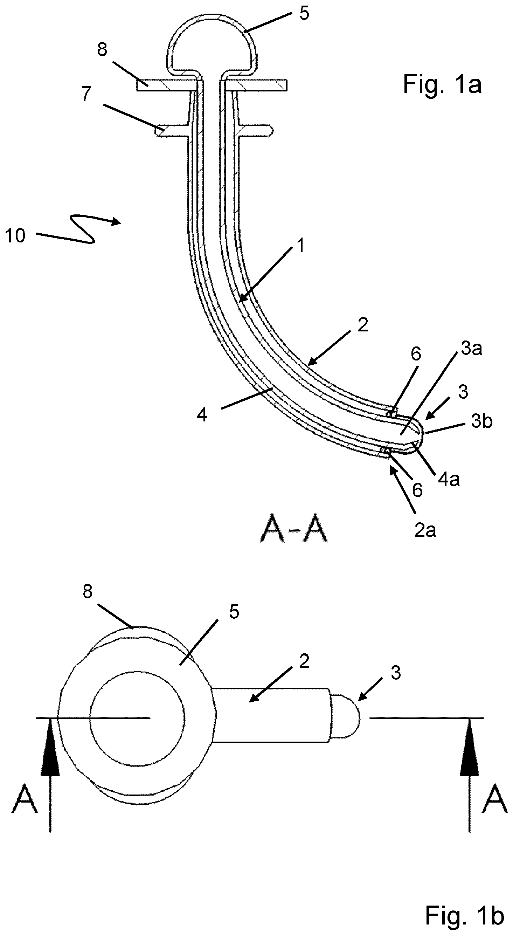

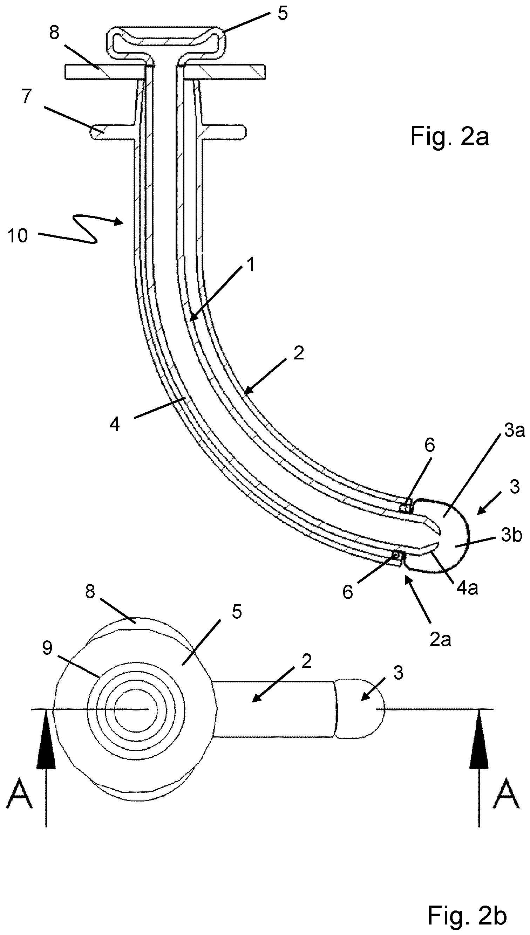

In den Figuren 1a, 1b, 2a und 2b sind verschiedene Ansichten eines erfindungsgemäßen Sets 10 mit einer erfindungsgemäßen Einführhilfe 1 und einer Tracheostomiekanüle 2 gezeigt. Die Einführhilfe 1 weist ein variables Fluidreservoir 5 auf, welches über einen Schlauch 4 mit einem Kopf 3 der Einführhilfe 1 verbunden ist. Über den Schlauch 4 kann ein Fluid von dem Fluidreservoir 5 in den Kopf 3 transportiert werden, und umgekehrt.

Zwecks Veranschaulichung ist der Innendurchmesser des Schlauches groß dargestellt, üblicherweise ist das Volumen des Schlauchs möglichst klein gewählt, um den Totraum des Schlauchs klein zu halten.

Der Kopf 3 weist eine flexible, dünnwandige Hülle 3b auf, die einen Hohlraum 3a in dem Kopf 3 umgibt. Der Hohlraum 3a dient der Aufnahme von Fluid aus dem Fluidreservoir 5. Befindet sich das Fluid in dem Fluidreservoir 5, wie es in den Figuren 1a und 1b gezeigt ist, ist der Außendurchmesser des Kopfes 3 kleiner als der Innendurchmesser der Tracheostomiekanüle 2. Der Kopf 3 kann auf diese Weise zusammen mit dem Schlauch 4 durch die proximale und distale Öffnung der Tracheostomiekanüle 2 geschoben werden.

In jeder der Ansichten 1a, 1b, 2a und 2b ist die Einführhilfe 1 derart in die Tracheostomiekanüle 2 eingeführt, dass der Kopf 3 aus dem distalen Ende 2a der Tracheostomiekanüle 2 herausragt, während das Fluidreservoir 5 auf der proximalen Seite angeordnet ist. Die Einführhilfe 1 kann jedoch auch getrennt von der Tracheostomiekanüle 2 aufbewahrt bzw. vermarktet werden. Das in den Figuren 1a, 1b, 2a und 2b gezeigte Kanülenschild 7 der Tracheostomiekanüle 2 ist ein optionales Merkmal zur Befestigung der Tracheostomiekanüle 2 an dem Hals eines Patienten.

Wird Fluid von dem Fluidreservoir 5 durch den Schlauch 4 in den Hohlraum 3a geleitet, dehnt sich der Kopf 3 aus bis er einen maximalen Außendurchmesser annimmt, der größer als der Innendurchmesser der Tracheostomiekanüle 2 ist. Der mit Fluid befüllte Kopf 3, wie er in den Figuren 2a und 2b gezeigt ist, kann in diesem Zustand nicht mehr durch das distale Ende der Tracheostomiekanüle 2 zurückgezogen werden.

Das variable Fluidreservoir 5 ist ein mit Fluid befüllter Ballon aus einem Elastomer, wie beispielsweise ein thermoplastisches Elastomer oder Gummi, und kann per Hand zusammengedrückt werden. Beim Zusammendrücken wird das Fluid aus dem Fluidreservoir 5 durch den Schlauch 4 in den Kopf 3 geleitet. Sinkt der Druck auf das Fluidreservoir 5, kehrt der Ballon in seinen Ausgangszustand zurück und das Fluid wird aus dem Kopf 3 zurück in das Fluidreservoir 5 geleitet. Eine von außen auf den Kopf 3 wirkende Kraft bewirkt den Transport von Fluid aus dem Kopf 3 in das Fluidreservoir 5, wenn der resultierende Druck im Kopf 3 größer als der Druck im Fluidreservoir 5 ist. Im Gebrauch der Einführhilfe 1 kann eine von außen auf den Kopf 3 wirkende Kraft durch einen Widerstand des zu durchstoßenden Tracheostomas und/oder der Trachea erzeugt werden. Eine Zunahme des Widerstands führt zu einer höheren auf den Kopf 3 wirkenden Kraft, die das in dem Kopf 3 befindliche Fluid durch den Schlauch 4 in das Fluidreservoir drängt. Der für das Zusammendrücken des Fluidreservoirs 5 erforderliche Kraftaufwand steigt, sodass ein Benutzer durch den steigenden Kraftaufwand auf einen erhöhten, auf den Kopf 3 wirkenden Widerstand schließen kann. Die Rückkopplung erlaubt es einem Benutzer insbesondere einen ungewöhnlich starken Widerstand zu erkennen und Verletzungen durch eine Reduzierung der auf das Fluidreservoir 5 ausgeübten Kraft zu vermeiden.

Um ein Zusammendrücken des Fluidreservoirs 5 mit einer Hand zu erleichtern, weist die Einführhilfe 1 an ihrem proximalen Ende einen Griff 8 auf. Der Griff 8 ist ein in radialer Richtung umlaufender Ring, der derart angeordnet ist, dass er mit mindestens zwei Fingern ergriffen werden kann, während mit dem Daumen der gleichen Hand ein Kraft auf das Fluidreservoir 8 ausgeübt werden kann. Der Griff 8 dient zugleich als Anschlag, der verhindert, dass die Einführhilfe 1 zu weit durch die Tracheostomiekanüle 2 hindurch geschoben werden kann. Das Fluidreservoir 5 hat in seinem befüllten Zustand annähernd eine Pilzform, wobei die Kuppel des Fluidreservoirs 5 von oben in distale Richtung eingedrückt werden kann. Das Fluidreservoir 5 weist weiter eine Vielzahl von konzentrisch zueinander angeordneten Verstärkungen 9 auf, die das Zusammenpressen der Hülle des Fluidreservoirs 5 in eine distale Richtung erleichtern und eine vorbestimmte Faltenbildung unterstützen. Die ringförmigen Verstärkungen 9 dienen auch als Markierung für die auf das Fluidreservoir 5 ausgeübte Kraft, die über das Fluid und den Kopf 3 auf eine Trachea oder ein Tracheostoma hydraulisch oder pneumatisch übertragbar ist.

Der Schlauch 4 hat ein distales Ende 4a, welches in den Hohlraum 3a des Kopfs 3 hineinragt. Das distale Ende 4a verjüngt sich in die distale Richtung und kann einen Teil der auf den Kopf 3 wirkenden Kraft aufnehmen. Auf diese Weise stabilisiert das distale Ende 4a des Schlauches 4 den Kopf 3.

Zusätzlich weist der Schlauch 4 in seinem distalen Bereich einen Abstandshalter 6 in Form eines umlaufenden Rings auf, der das distale Ende 4a des Schlauches 4 in dem Lumen der Tracheostomiekanüle 2 zentriert. Die Zentrierung unterstützt eine gezielte Positionierung der Einführhilfe 3 und insbesondere des Kopfes 3 zum Durchstoßen eines Tracheostomas.

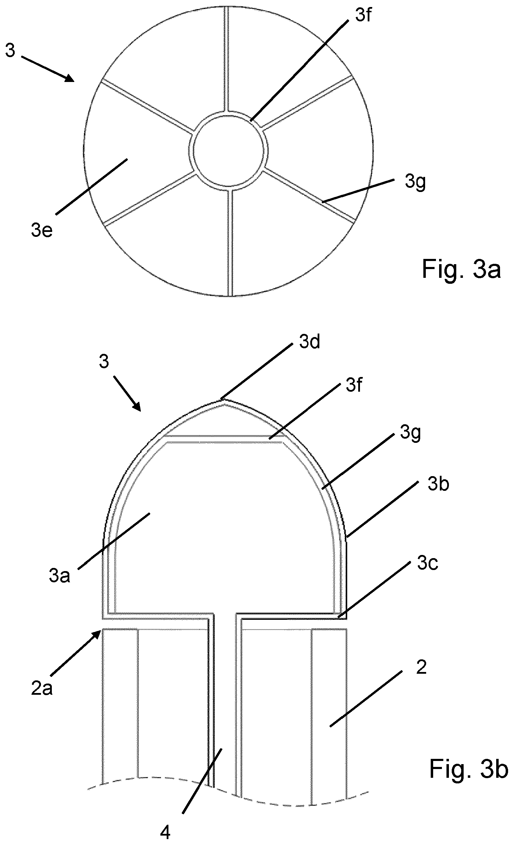

In den Figuren 3a und 3b ist eine Detailansicht eines Kopfs 3 einer Einführungshilfe 1 gemäß einer weiteren Ausführungsform der vorliegenden Erfindung gezeigt, wobei in der Figur 3b ein Querschnitt des Kopfs 3 und in der Figur 3a eine Draufsicht auf das distale Ende des Kopfs 3 jeweils in einem befüllten Zustand gezeigt ist.

Der Kopf 3 besteht aus einer flexiblen, dünnwandigen Hülle 3b, die einen Hohlraum 3a umgibt. Der Hohlraum 3a ist über einen Schlauch 4 mit einem Fluidreservoir 5 verbunden (in der Figur 3b nur angedeutet). In seinem mit Fluid befüllten Zustand hat der Kopf 3 annähernd die Form eines Kegels mit einer Basis 3c, einer Spitze 3d und einer sich von der Basis 3c zur Spitze 3d hin verjüngenden, umlaufenden Seitenwand 3e, wobei die Basis 3c näher an dem distalen Ende 2a Tracheostomiekanüle 2 als die Spitze 3d angeordnet ist. Die Basis 3c bildet einen maximalen Außendurchmesser des Kopfs 3 der größer als der Innendurchmesser der Tracheostomiekanüle 2 ist.

Weiter hat der Kopf lokale Verstärkungen, die eine gezielte Faltenbildung des Kopfes 3 ermöglichen, wenn das Fluid aus dem Kopf 3 entfernt wird. Die lokalen Verstärkungen helfen die im Gebrauch der Einführhilfe 1 auf den Kopf 3 vor allem in axialer Richtung einwirkenden Kräfte aufzunehmen und stabilisieren so die Form des Kopfs 3.

Die lokalen Verstärkungen umfassen in der Seitenwand 3e von der Spitze 3d zur Basis 3c verlaufende Rippen 3g, wobei im Inneren des die Spitze 3d umgebenden, zentralen Verstärkungsringes 3f keine weiteren Verstärkungen mehr vorliegen, so dass die Spitze leichter verformbar ist, was wiederum über das Fluid eine Rückmeldung im Fluidreservoir schon bei entsprechend geringen auf die Spitze wirkenden Kräften bewirkt. Der Verstärkungsring 3f hat einen kleineren Au ßendurchmesser als der Innendurchmesser der Tracheostomiekanüle 2.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarerer Merkmalskombinationen wird hier nur der Kürze und der Lesbarkeit der Beschreibung wegen verzichtet.

Während die Erfindung im Detail in den Zeichnungen und der vorangehenden Beschreibung dargestellt und beschreiben wurde, erfolgt diese Darstellung und Beschreibung lediglich beispielhaft und ist nicht als Beschränkung des Schutzbereichs gedacht, so wie er durch die Ansprüche definiert wird. Die Erfindung ist nicht auf die offenbarten Ausführungsformen beschränkt.

Abwandlungen der offenbarten Ausführungsformen sind für den Fachmann aus den Zeichnungen, der Beschreibung und den beigefügten Ansprüchen offensichtlich. In den Ansprüchen schließt das Wort "aufweisen" nicht andere Elemente oder Schritte aus, und der unbestimmte Artikel "eine" oder "ein" schließt eine Mehrzahl nicht aus. Die bloße Tatsache, dass bestimmte Merkmale in unterschiedlichen Ansprüchen beansprucht sind, schließt ihre Kombination nicht aus. Bezugszeichen in den Ansprüchen sind nicht als Beschränkung des Schutzbereichs gedacht.

### Bezugszeichenliste

- 1: Einführhilfe
- 2: Tracheostomiekanüle
- 2a: distales Ende der Tracheostomiekanüle
- 3: Kopf
- 3a: Hohlraum
- 3b: Hülle
- 3c: Basis
- 3d: Spitze
- 3e: Seitenwand
- 3f: Verstärkungsring
- 3g: Verstärkungsrippen
- 4: Schlauch
- 4a: distale Ende des Schlauches 4
- 5: Fluidreservoir
- 6: Abstandhalter
- 7: Kanülenschild
- 8: Griff der Einführhilfe
- 9: ringförmige Verstärkungen des Fluidreservoirs 5
- 10: Set mit einer Einführhilfe 1 und einer Tracheostomiekanüle 2

## Patentansprüche

1. Einführhilfe (1) für eine Tracheostomiekanüle (2), mit einem durch die Tracheostomiekanüle (2) hindurchführbaren Kopf (3), , wobei der Kopf (3) der Einführhilfe (1) hohl ist und der Hohlraum (3a) des Kopfes (3) über einen durch das Lumen der Tracheostomiekanüle (2) verlaufenden Schlauch (4) mit einem variablen Fluidreservoir (5) zum Befüllen und Entleeren des Hohlraums (3a) verbunden ist, das im Gebrauch auf der proximalen Seite der Tracheostomiekanüle (2) angeordnet ist, **dadurch gekennzeichnet, dass** der Kopf (3) im Gebrauch aus dem distalen Ende (2a) der Tracheostomiekanüle (2) herausragt und der das Fluidreservoir (5) und den Kopf (3) verbindende Schlauch (4) abschnittsweise in den Hohlraum (3a) des Kopfes (3) hineinragt, wobei sich das in den Hohlraum (3a) hineinragende Ende (4a) des Schlauches (4) in distale Richtung verjüngt, wobei der Kopf (3) eine dünnwandige, flexible Hülle (3b) aufweist, so dass der Kopf (3) bei gefülltem Hohlraum (3a) gegenüber der Längsachse der Tracheostomiekanüle (2) einen maximalen Außendurchmesser annimmt, der größer als der Innendurchmesser der Tracheostomiekanüle (2) ist, und bei entleertem Hohlraum (3b) gegenüber der Längsachse der Tracheostomiekanüle (2) einen maximalen Außendurchmesser annehmen kann, der kleiner ist als der Innendurchmesser der Tracheostomiekanüle (2).

2. Einführhilfe (1) nach Anspruch 1, **dadurch gekennzeichnet, dass** das variable Fluidreservoir (5), der Schlauch (4) und der Kopf (3) ein geschlossenes Volumen bilden.

3. Einführhilfe (1) nach Anspruch 1 oder 2, **dadurch gekennzeichnet, dass** das maximale Volumen des variablen Fluidreservoirs (5) dem 0,5-fachen bis 5-fachen des maximalen Volumens des Kopfs (3) entspricht, wobei vorzugsweise das maximale Volumen des variablen Fluidreservoirs (5) und das maximale Volumen des Kopfes (3) gleich groß sind.

4. Einführhilfe (1) nach einem der Ansprüche 1 bis 3, **dadurch gekennzeichnet, dass** der den maximalen Durchmesser definierende Abschnitt des Kopfes (3) im gefüllten Zustand näher an dem distalen Ende (2a) der Tracheostomiekanüle (2) angeordnet ist als an dem distalen Ende des Kopfes (3).

5. Einführhilfe (1) nach einem der Ansprüche 1 bis 4, **dadurch gekennzeichnet, dass** der flexible Kopf (3) im gefüllten Zustand annähernd die Form eines Kegels mit einer Basis (3c), einer Spitze (3d) und einer sich von der Basis (3c) zur Spitze (3d) hin verjüngenden, umlaufenden Seitenwand (3e) hat, wobei die Basis (3c) näher an dem distalen Ende (2a) der Tracheostomiekanüle (2) als die Spitze (3d) angeordnet ist.

6. Einführhilfe (1) nach einem der Ansprüche 1 bis 5, **dadurch gekennzeichnet, dass** der Kopf (3) lokale Verstärkungen zur Erzeugung einer gezielten Faltenbildung des Kopfes (3) hat, wenn das Fluid aus dem Kopf (3) abgezogen wird.

7. Einführhilfe (1) nach Anspruch 6, **dadurch gekennzeichnet, dass** die Verstärkungen in der Seitenwand (3e) von der Spitze (3d) zur Basis (3c) verlaufende Rippen (3f) sind, wobei die Rippen (3f) vorzugsweise an einem die Spitze (3d) des Kopfs (3) umgebenden zentralen Verstärkungsring (3g) enden, dessen Außendurchmesser kleiner als der Innendurchmesser der Tracheostomiekanüle (2) ist.

8. Einführhilfe (1) nach einem der Ansprüche 1 bis 7, **dadurch gekennzeichnet, dass** das Fluidreservoir (5) ein von Hand zusammendrückbarer, elastischer Ballon ist.

9. Einführhilfe (1) nach einem der Ansprüche 1 bis 8, **dadurch gekennzeichnet, dass** der das Fluidreservoir (5) und den Kopf (3) verbindende Schlauch (4) aus einem härteren Material besteht als die Hülle (3b) des Kopfs (3).

10. Einführhilfe (1) nach einem der Ansprüche 1 bis 9, **dadurch gekennzeichnet, dass** das maximale Füllvolumen des Fluidreservoirs von 0,3 ml bis 5 ml und/oder das maximale Füllvolumen des Kopfs von 0,1 ml bis 3 ml beträgt.

11. Einführhilfe (1) nach einem der Ansprüche 1 bis 10, **dadurch gekennzeichnet, dass** die flexible Hülle (3b) des Kopfes (3) aus einem unelastischen Material besteht oder aus einem Elastomer besteht, wobei das Elastomer vorzugsweise eine Shore-A-Härte in einem Bereich von 5 bis 70 aufweist, wobei der Elastomer vorzugsweise ein thermpolastischer Elastomer ausgewählt aus der Gruppe thermoplastischer Styrol-Blockcopolymere, thermoplastischer Polyamidelastomere, Polyurethan, Ethylen-Vinylacetat-Copolymer, oder Silikon ist.

12. Einführhilfe (1) nach einem der Ansprüche 1 bis 11, **dadurch gekennzeichnet, dass** die flexible Hülle (3b) des Kopfes (3) eine mittlere Wandstärke zwischen 5 µm und 1 mm, vorzugsweise zwischen 10 µm und 500 µm.

13. Einführhilfe (1) nach einem der Ansprüche 1 bis 12, **dadurch gekennzeichnet, dass** der Kopf (3) einen Kanal zum Hindurchführen eines Seldingerdrahts und/oder eines Führungskatheters aufweist.

14. Einführhilfe (1) nach einem der Ansprüche 1 bis 13, **dadurch gekennzeichnet, dass** das Fluid eine inkompressible Flüssigkeit, vorzugsweise Wasser, oder ein komprimierbares Gas, vorzugsweise Luft, ist.

15. Set (10) mit einer Tracheostomiekanüle (2) und einer Einführhilfe (1) nach einem der Ansprüche 1 bis 14, wobei die Einführhilfe (1) und die Tracheostomiekanüle (2) derart dimensioniert sind, dass der Kopf (3) und der den Kopf (3) mit dem variablen Fluidreservoir (5) verbindende Schlauch (4) durch die Tracheostomiekanüle (2) hindurchführbar sind und das Fluidreservoir (5) auf der proximalen Seite der Tracheostomiekanüle (2) angeordnet ist, **dadurch gekennzeichnet, dass** die Einführhilfe (1) und die Tracheostomiekanüle (2) derart dimensioniert sind, dass der Kopf (3) aus der distalen Öffnung der Tracheostomiekanüle (2) herausragt und der das Fluidreservoir (5) und den Kopf (3) verbindende Schlauch (4) abschnittsweise in den Hohlraum (3a) des Kopfes (3) hineinragt, wobei sich das in den Hohlraum (3a) hineinragende Ende (4a) des Schlauches (4) in distale Richtung verjüngt, und wobei der Kopf (3) bei gefülltem Hohlraum (3a) gegenüber der Längsachse der Tracheostmoiekanüle (2) einen maximalen Außendurchmesser annimmt, der größer als der Innendurchmesser der Tracheostomiekanüle (2) ist, und bei entleertem Hohlraum (3a) gegenüber der Längsachse der Tracheostomiekanüle (2) einen maximalen Außendurchmesser annehmen kann, der kleiner ist als der Innendurchmesser der Tracheostomiekanüle (2).

## Claims

1. Insertion aid (1) for a tracheostomy tube (2), with a head (3) which can be guided through the tracheostomy tube (2), wherein the head (3) of the insertion aid (1) is hollow and the cavity (3a) of the head (3) is connected via a tube (4) running through the lumen of the tracheostomy tube (2) to a variable fluid reservoir (5) for filling and emptying the cavity (3a), which is arranged on the proximal side of the tracheostomy tube (2) in use, **characterized in that** the head (3) protrudes from the distal end (2a) of the tracheostomy tube (2) in use and the tube (4) connecting the fluid reservoir (5) and the head (3) extends into the cavity (3a) of the head (3) in sections, wherein the end (4a) of the tube (4) extending into the cavity (3a) narrows in the distal direction, wherein the head (3) has a thin-walled, flexible sheath (3b), so that, when the cavity (3a) is filled, the head (3) takes on a maximum outside diameter with respect to the longitudinal axis of the tracheostomy tube (2), which is larger than the inside diameter of the tracheostomy tube (2), and, when the cavity (3b) is emptied, can take a maximum outside diameter with respect to the longitudinal axis of the tracheostomy tube (2), which is smaller than the inside diameter of the tracheostomy tube (2).

2. Insertion aid (1) according to claim 1, **characterized in that** the variable fluid reservoir (5), the tube (4) and the head (3) form a closed volume.

3. Insertion aid (1) according to claim 1 or 2, **characterized in that** the maximum volume of the variable fluid reservoir (5) corresponds to 0.5 times to 5 times the maximum volume of the head (3), wherein the maximum volume of the variable fluid reservoir (5) and the maximum volume of the head (3) are preferably equal.

4. Insertion aid (1) according to one of claims 1 to 3, **characterized in that** the section of the head (3) defining the maximum diameter in the filled state is arranged closer to the distal end (2a) of the tracheostomy tube (2) than the distal end of the head (3).

5. Insertion aid (1) according to one of claims 1 to 4, **characterized in that** the flexible head (3) has approximately the shape of a cone with a base (3c), a tip (3d) and a circumferential side wall (3e) narrowing from the base (3c) to the tip (3d) in the filled state, wherein the base (3c) is arranged closer to the distal end (2a) of the tracheostomy tube (2) than the tip (3d).

6. Insertion aid (1) according to one of claims 1 to 5, **characterized in that** the head (3) has local reinforcements for generating a targeted wrinkling of the head (3) when the fluid is withdrawn from the head (3).

7. Insertion aid (1) according to claim 6, **characterized in that** the reinforcements in the side wall (3e) are ribs (3f) running from the tip (3d) to the base (3c), wherein the ribs (3f) preferably end at a central reinforcing ring (3g) surrounding the tip (3d) of the head (3) the outside diameter of which is smaller than the inside diameter of the tracheostomy tube (2).

8. Insertion aid (1) according to one of claims 1 to 7, **characterized in that** the fluid reservoir (5) is an elastic balloon compressible by hand.

9. Insertion aid (1) according to one of claims 1 to 8, **characterized in that** the tube (4) connecting the fluid reservoir (5) and the head (3) consists of a harder material than the sheath (3b) of the head (3).

10. Insertion aid (1) according to one of claims 1 to 9, **characterized in that** the maximum filling volume of the fluid reservoir is from 0.3 ml to 5 ml and/or the maximum filling volume of the head is from 0.1 ml to 3 ml.

11. Insertion aid (1) according to one of claims 1 to 10, **characterized in that** the flexible sheath (3b) of the head (3) consists of an inelastic material or consists of an elastomer, wherein the elastomer preferably has a Shore A hardness in a range of from 5 to 70, wherein the elastomer is preferably a thermoplastic elastomer selected from the group of thermoplastic styrene block copolymers, thermoplastic polyamide elastomers, polyurethane, ethylene-vinyl acetate copolymer, or silicone.

12. Insertion aid (1) according to one of claims 1 to 11, **characterized in that** the flexible sheath (3b) of the head (3) [has] an average wall thickness between 5 µm and 1 mm, preferably between 10 µm and 500 µm.

13. Insertion aid (1) according to one of claims 1 to 12, **characterized in that** the head (3) has a channel for guiding a Seldinger guide wire and/or a guide catheter through.

14. Insertion aid (1) according to one of claims 1 to 13, **characterized in that** the fluid is an incompressible liquid, preferably water, or a compressible gas, preferably air.

15. Set (10) with a tracheostomy tube (2) and an insertion aid (1) according to one of claims 1 to 14, wherein the insertion aid (1) and the tracheostomy tube (2) are dimensioned such that the head (3) and the tube (4) connecting the head (3) to the variable fluid reservoir (5) can be guided through the tracheostomy tube (2) and the fluid reservoir (5) is arranged on the proximal side of the tracheostomy tube (2), **characterized in that** the insertion aid (1) and the tracheostomy tube (2) are dimensioned such that the head (3) protrudes from the distal opening of the tracheostomy tube (2) and the tube (4) connecting the fluid reservoir (5) and the head (3) extends into the cavity (3a) of the head (3) in sections, wherein the end (4a) of the tube (4) extending into the cavity (3a) narrows in the distal direction, and wherein, when the cavity (3a) is filled, the head (3) takes on a maximum outside diameter with respect to the longitudinal axis of the tracheostomy tube (2), which is larger than the inside diameter of the tracheostomy tube (2), and, when the cavity (3a) is emptied, can take a maximum outside diameter with respect to the longitudinal axis of the tracheostomy tube (2), which is smaller than the inside diameter of the tracheostomy tube (2).

## Revendications

1. Aide à l'insertion (1) pour une canule trachéostomique (2), comprenant une tête (3) pouvant être passée par la canule trachéostomique (2), la tête (3) de l'aide à l'insertion (1) étant creuse et la cavité (3a) de la tête (3) étant reliée, par un tuyau flexible (4) passant par le lumen de la canule trachéostomique (2), à un réservoir variable de fluide (5) destiné au remplissage et au vidage de la cavité (3a) et disposé, lors de l'utilisation, du côté proximal de la canule trachéostomique (2), **caractérisée en ce que**, lors de l'utilisation, la tête (3) dépasse de l'extrémité distale (2a) de la canule trachéostomique (2) et le tuyau flexible (4) reliant le réservoir de fluide (5) à la tête (3) s'étend en partie jusque dans la cavité (3a) de la tête (3), l'extrémité (4a) du tuyau flexible (4), qui s'étend jusque dans la cavité (3a), s'amenuisant dans la direction distale, la tête (3) comprenant une enveloppe (3b) à paroi mince et flexible, de sorte que la tête (3), lorsque la cavité (3a) est remplie, ait un diamètre extérieur maximal par rapport à l'axe longitudinal de la canule trachéostomique (2), qui est supérieur au diamètre intérieur de la canule trachéostomique (2), et puisse avoir, lorsque la cavité (3b) est vidée, un diamètre extérieur maximal par rapport à l'axe longitudinal de la canule trachéostomique (2), qui est inférieur au diamètre intérieur de la canule trachéostomique (2).

2. Aide à l'insertion (1) selon la revendication 1, **caractérisée en ce que** le réservoir variable de fluide (5), le tuyau flexible (4) et la tête (3) forment un volume fermé.

3. Aide à l'insertion (1) selon la revendication 1 ou 2, **caractérisée en ce que** le volume maximal du réservoir variable de fluide (5) est de 0,5 à 5 fois plus important que le volume maximal de la tête (3), le volume maximal du réservoir variable de fluide (5) et le volume maximal de la tête (3) étant égaux.

4. Aide à l'insertion (1) selon l'une des revendications 1 à 3, **caractérisée en ce que** la section de la tête (3) définissant le diamètre maximum est disposée, à l'état rempli, plus près de l'extrémité distale (2a) de la canule trachéostomique (2) que de l'extrémité distale de la tête (3).

5. Aide à l'insertion (1) selon l'une des revendications 1 à 4, **caractérisée en ce que** la tête flexible (3) a, à l'état rempli, la forme se rapprochant de celle d'un cône ayant une base (3c), un sommet (3d) et une paroi latérale (3e) périphérique allant, en se rétrécissant, de la base (3c) au sommet (3d), la base (3c) étant disposée plus près de l'extrémité distale (2a) de la canule trachéostomique (2) que le sommet (3d).

6. Aide à l'insertion (1) selon l'une des revendications 1 à 5, **caractérisée en ce que** la tête (3) a des renforts locaux pour produire un pliage ciblé de la tête (3), le fluide étant tiré de la tête.

7. Aide à l'insertion (1) selon la revendication 6, **caractérisée en ce que** les renforts sont des nervures (3f) passant dans la paroi latérale (3e) du sommet (3d) à la base (3c), les nervures se terminant de préférence à un anneau de renfort (3g) central encerclant le sommet (3d) de la tête (3), anneau dont le diamètre extérieur est plus petit que le diamètre intérieur de la canule trachéostomique (2).

8. Aide à l'insertion (1) selon l'une des revendications 1 à 7, **caractérisée en ce que** le réservoir de fluide (5) est un ballon élastique pouvant être pressé à la main.

9. Aide à l'insertion (1) selon l'une des revendications 1 à 8, **caractérisée en ce que** le tuyau flexible (4) reliant le réservoir de fluide (5) et la tête (3) est constitué d'un matériau plus dur que l'enveloppe (3b) de la tête (3).

10. Aide à l'insertion (1) selon l'une des revendications 1 à 9, **caractérisée en ce que** le volume de remplissage maximum du réservoir de fluide est de 0,3 ml à 5 ml et/ou que le volume de remplissage maximum de la tête est de 0,1 ml à 3 ml.

11. Aide à l'insertion (1) selon l'une des revendications 1 à 10, **caractérisée en ce que** l'enveloppe (3b) flexible de la tête (3) est constituée d'un matériau non élastique ou d'un élastomère, l'élastomère ayant de préférence une dureté shore A dans une plage de 5 à 70, l'élastomère étant de préférence un élastomère thermoplastique choisi dans le groupe des copolymères à bloc de styrène thermoplastiques, des élastomères de polyamide thermoplastiques, du polyuréthane, du copolymère d'éthylène et d'acétate de vinyle ou du silicone.

12. Aide à l'insertion (1) selon l'une des revendications 1 à 11, **caractérisée en ce que** l'enveloppe flexible (3b) de la tête (3) a une épaisseur de paroi moyenne comprise entre 5 µm et 1 mm, de préférence entre 10 µm et 500 µm.

13. Aide à l'insertion (1) selon l'une des revendications 1 à 12, **caractérisée en ce que** la tête (3) comprend un canal pour faire passer un fil Seldinger et/ou un cathéter de guidage.

14. Aide à l'insertion (1) selon l'une des revendications 1 à 13, **caractérisée en ce que** le fluide est un liquide incompressible, de préférence de l'eau, ou un gaz pouvant être comprimé, de préférence de l'air.

15. Ensemble (10) comprenant une canule trachéostomique (2) et une aide à l'insertion (1) selon l'une des revendications 1 à 14, l'aide à l'insertion (1) et la canule trachéostomique (2) étant dimensionnées de manière telle que la tête (3) et le tuyau flexible (4) reliant la tête (3) au réservoir variable de fluide (5) puissent être passés par la canule trachéostomique (2), et que le réservoir de fluide (5) soit disposé sur le côté proximal de la canule trachéostomique (2), **caractérisé en ce que** l'aide à l'insertion (1) et la canule trachéostomique (2) sont dimensionnées de manière telle que la tête (3) dépasse de l'ouverture distale de la canule trachéostomique (2) et que le tuyau flexible (4) reliant le réservoir de fluide (5) à la tête (3) s'étende, par sections, jusque dans la cavité (3a) de la tête (3), l'extrémité (4a) du tuyau flexible (4) s'étendant jusque dans la cavité (3a) s'amincissant dans la direction distale, et la tête (3) prenant, lorsque la cavité (3a) est remplie, par rapport à l'axe longitudinal de la canule trachéostomique (2), un diamètre extérieur maximal qui est plus grand que le diamètre intérieur de la canule trachéostomique (2), et, lorsque la cavité (3a) est vidée, pouvant prendre, par rapport à l'axe longitudinal de la canule trachéostomique (2), un diamètre extérieur maximal qui est plus petit que le diamètre intérieur de la canule trachéostomique (2).
